# EUROPÄISCHE PATENTSCHRIFT

(11) **EP 0 456 120 B1**
(45) Veröffentlichungstag und Bekanntmachung des Hinweises auf die Patenterteilung: **29.03.1995**
(21) Anmeldenummer: 91107155.3
(22) Anmeldetag: 03.05.1991
(51) Int. Cl.: A61B 17/60, A61B 17/58

(54) **Kraftübertragungsvorrichtung bzw. Fixationsvorrichtung für osteosynthetische Arbeiten**
Power transmission device or fixation device for osteosynthetic-work
Dispositif de transmission de puissance ou encore dispositif de fixation pour travaux d'ostéosynthèse

(30) Priorität: 10.05.1990 DE 4014973
(43) Veröffentlichungstag der Anmeldung: 13.11.1991
(73) Patentinhaber: Härle, Anton, Dr., D-48161 Münster (DE)
(72) Erfinder: Härle, Anton, Dr., D-48161 Münster (DE)
(74) Vertreter: Habbel, Hans-Georg, Dipl.-Ing.

(56) Entgegenhaltungen:
- EP-A- 0 073 455
- WO-A-87/01026
- GB-A- 2 110 094
- GB-A- 2 250 417
- US-A- 2 333 033

## Beschreibung

Die Erfindung betrifft eine Kraftübertragungsvorrichtung bzw. Fixationsvorrichtung gemäß dem Oberbegriff des Hauptanspruches, wobei die beiden Elemente an Osteosynthesehilfsmitteln oder dem Knochen direkt angreifen.

In der Osteosynthese von Röhrenknochen nach Frakturen oder Korrekturosteotomien gibt es regelmäßig das Erfordernis, die Knochen bzw. Knochenfragmente in eine bestimmte Stellung und Ausrichtung zueinander zu bringen und dann in dieser Position solange ruhigzustellen, bis eine knöcherne Durchbauung erreicht ist. Während die Aufgabe der Fragmentfixation und interfragmentären Fragmentkompression schon seit langem große Aufmerksamkeit geschenkt worden war, ist man der Aufgabe, die Knochen bzw. Knochenfragmente in eine bestimmte Stellung und Ausrichtung zueinander zu bringen, lange Zeit ausgewichen.

Die manuelle Geschicklichkeit des Operateurs hatte diese Problematik zu beherrschen, was ohne entsprechende technische Ausrüstung nicht selten zu unbefriedigenden Ergebnissen führte. Lange Operationszeiten, unvollständige Repositionen nach Frakturen mit Achsfehlstellungen mußten in Kauf genommen werden, wobei letztere ihre Konsequenzen in posttraumatischen Gelenkschädigungen infolge der Inkongruenz der Gelenkpartner bzw. lokaler Knorpelüberlastungen hatten.

Eine Korrektur der Fragmenteinstellung war früher nur mit Hilfe äußerer Spannsysteme möglich, die über eine Distraktion die Fragmentreposition auch mit nachträglicher Achskorrektur ermöglichten. Diese Systeme wiesen aber den Nachteil der äußeren Fixation auf, d. h. es ragten immer Gewindestifte, die im Knochen verankert waren, aus der Haut heraus, und Infektionen entlang dieser Stifte traten in großer Häufigkeit auf. Heute wird eine derartige äußere Fixation nur noch für die Primärversorgung von Schwerstverletzten für wenige Tage als angemessen erachtet.

Kraftübertragungsvorrichtungen für osteosynthetische Arbeiten sind allgemein z. B. aus der DE-A-27 08 866 und der DE-A-29 38 202 bekannt.

Mit der Kraftübertragungsvorrichtung gemäß der gattungsbildenden EP-B-0 073 455 wurde dann ein Repositions-Instrumentarium für die interne Osteosynthese bereitgestellt. Mit seiner Hilfe konnten Fragmente kontrolliert distrahiert werden und so eine für die Korrektur grundlegende Voraussetzung, nämlich die Überwindung der Weichteilspannung, technisch verwirklicht werden. Auch die Seitenverschiebung der Fragmente konnte korrigiert werden, allerdings mit dem Nachteil, daß dabei die reponierende Schraube in die der Plattenseite gegenüberliegenden Weichteile eindringen mußte und hier zu Verletzungen von Gefäßen und Nerven führen konnte.

Ein wichtiges Erfordernis bei der Frakturreposition, das insbesondere bei Wirbelfrakturen, aber auch bei Korrekturosteotomien auftritt, nämlich die dosierte und kontrollierte Winkelkorrektur war aber mit diesem Gerät nicht möglich. Die Fixation auf der Osteosyntheseplatte hatte zwar für Verlängerungs-Osteomien zu einer bedeutsamen Vereinfachung des Operationsverfahrens geführt, interferierte aber mit dem Erfordernis einer Plattenanbiegung zur besseren Adaptation an die anatomischen Verhältnisse.

Bei der Behandlung von Wirbelsäulendeformitäten und Wirbelkörperfrakturen ist wegen der besonderen anatomischen Verhältnisse (es steht nur wenig Raum für die Schraubenfixation wegen des Rückenmarks zur Verfügung; jeweils nur eine Schraube je Wirbelkörper) eine Korrektur der Fragmente vor der Plattenfixation wünschenswert. Auch kann das Vorbohren und Eindrehen von Schrauben nicht ohne weiteres durch eine schon aufliegende Platte (wie z. B. bei der Extremitätenosteosynthese) vorgenommen werden, wenn man nicht Gefahr laufen will, wegen mangelndem Überblick über die anatomischen Verhältnisse Rückenmarksschädigungen auszulösen. Auch bei anspruchsvollen Korrekturosteomien an den Extremitäten, z. B. in Gelenknähe kann es sehr vorteilhaft sein, wenn die neue Einstellung ohne das Erfordernis einer Plattenfixation überprüft und getestet werden kann. Die Beurteilung des Operationsergebnisses und eventuell erforderliche Nachkorrekturen sind so leichter umzusetzen, wobei vor allem das Umsetzen von Schrauben, das manchmal erhebliche Probleme beinhaltet, entfallen kann.

Der Erfindung liegt somit die Aufgabe zugrunde, Knochen bzw. Knochenfragmente in eine bestimmte Stellung und Ausrichtung zueinander zu bringen und die gattungsbildende Kraftübertragungsvorrichtung so zu verbessern, daß neben der Distraktion und Verkürzung auch andere Korrekturen im Raum dosiert vorgenommen werden können, d. h. daß damit kontrolliert Winkelveränderungen der Osteosynthese - Fixationselemente bzw. der Knochen möglich werden.

Diese der Erfindung zugrundeliegende Aufgabe wird durch die Lehre des Hauptanspruches gelöst.

Vorteilhafte Ausgestaltungen sind in den Unteransprüchen erläutert. Insbesondere wird durch die Lehre des Hauptanspruches in Verbindung mit dem Anspruch 3 die Möglichkeit gegeben, Winkelkorrekturen auch in drei Ebenen vorzunehmen.

Aus der WO 87/01026 eine Fixiervorrichtung für die Wirbelsäulenchirurgie bekanntgeworden, bei der aber keine kontrolliert antreibbare Schwenkung des den Knochenpin od. dgl. tragenden Bauteiles möglich ist.

Aus der GB-A-21 10 094 ist eine Fixiervorrichtung bekanntgeworden, bei der zwar eine Schwenkung des die Knochenpins od. dgl. tragenden Bauteiles möglich ist, wobei aber das Zentrum der bogenförmigen Führungsschiene in dem Knochen selbst liegt. Daher ist die Einstellung durch einen Chirurgen sehr schwierig. Die Translation des Bauteiles auf einer Führungsschiene führt auch zu einer erheblichen Baulänge der Vorrichtung, die die notwendige Miniaturisierung nicht zuläßt.

Mit anderen Worten ausgedrückt wird vorgeschlagen, daß die gattungsbildende Kraftübertragungsvorrichtung zu Repositions- und Fragmentausrichtungen ausgebaut wird, derart, daß z. B. bei der Behandlung von Wirbelsäulendeformitäten und Wirbelkörperfrakturen, eine Korrektur der Fragmente auch vor der Plattenfixation möglich ist, und zwar in allen den dabei erforderlichen Ebenen.

Ausführungsbeispiele der Erfindung werden nachfolgend anhand der Zeichnungen erläutert. Die Zeichnungen zeigen dabei in
- Fig. 1: eine Ausführungsform der Vorrichtung, in
- Fig. 2: eine abgeänderte Ausführungsform für den Antrieb des z. B. radartigen Körpers, in
- Fig. 3: schaubildlich eine Vorrichtung mit angeschlossenem Zusatzteil, in
- Fig. 4: einen Schnitt durch das angeschlossene Zusatzteil, in
- Fig. 5: in einer auseinandergezogenen Darstellungsweise eine abgeänderte Ausführungsform, in
- Fig. 6: eine weitere abgeänderte Ausführungsform, in
- Fig. 7: einen Schnitt durch die Ausführungsform gemäß Fig. 6 gemäß der Linie 7 - 7 in Fig. 8, in
- Fig. 8: einen Schnitt gemäß der Linie 8 - 8 in Fig. 7 und in
- Fig. 9: eine weitere abgeänderte Ausführungsform.

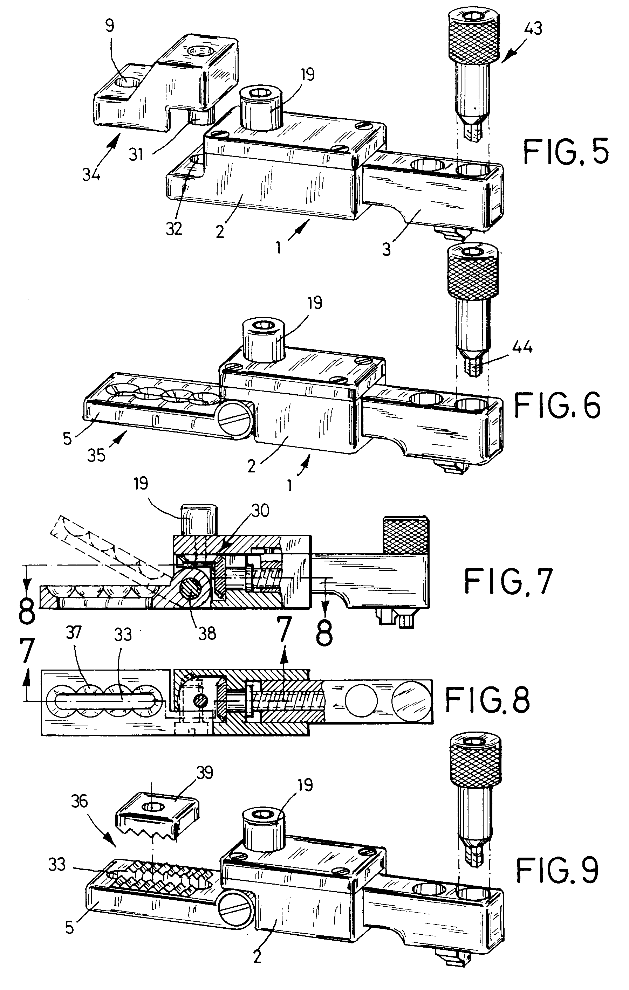

In den Zeichnungen ist eine Kraftübertragungs- bzw. Fixationsvorrichtung 1 dargestellt, die hinsichtlich ihres Grundaufbaues im wesentlichen wie in der gattungsbildenden EP-B-0 073 455 ausgebildet ist, d. h. aus zwei Spannelementen 2 und 3 besteht, wobei das Spannelement 2 gehäuseartig ausgebildet ist und einen als Beispiel in Fig. 7 und 8 dargestellten Antrieb 30 aufnimmt, durch den das Spannelement 3 gegenüber dem Spannelement 2 in Längsrichtung bewegt werden kann. Der für den Betrieb dieses Antriebes nach außen weisende Innensechskant ist bei 19 erkennbar.

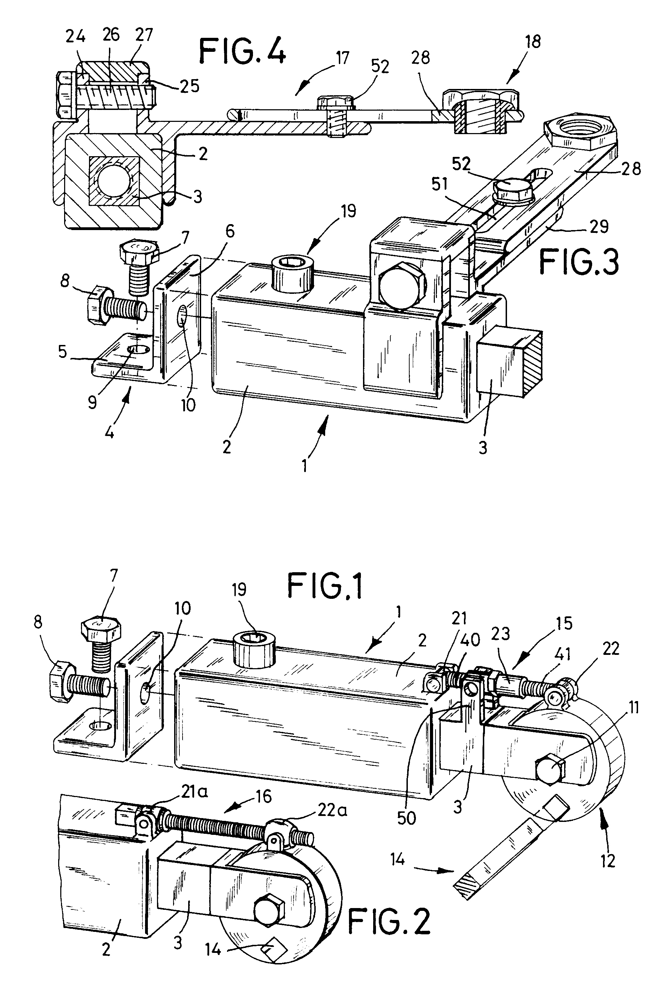

Um die Kraftübertragungsvorrichtung 1 ortsfest festzulegen, ist ein Befestigungsmittel 4 bzw. 34, 35 bzw. 36 vorgesehen, das als Winkelstück ausgebildet ist und gemäß Fig. 3 und 4 einen Bodenflansch 5 und einen Vertikalflansch 6 aufweist, wobei beide Flansche Bohrungen 9 bzw. 10 besitzen, durch die Haltemittel 7, 8 eingeführt werden können. Während normalerweise das Haltemittel 8, das zur Festlegung des Vertikalflansches 6 an der Rückseite des Spannelementes 2 dient, als Schraube mit Maschinengewinde ausgebildet ist, kann das Haltemittel 7 z. B. als Knochenschraube, Gewindepin od. dgl. ausgebildet sein, d. h. die Kraftübertragungsvorrichtung 1 kann beispielsweise an einer nicht dargestellten Osteosynthesespannplatte festgelegt werden, kann aber auch ohne Fixation an einer Platte eingesetzt werden, d. h. ortsfest dadurch festgelegt werden, daß ein Angriff gegen verschiedene Widerlager, wie Knochenschrauben, Stifte, Stäbe oder Haken erfolgt. Das mit Maschinengewinde ausgerüstete Haltemittel 7 ist also nur beispielhaft dargestellt.

Aus der Erläuterung des Befestigungsmittels 4 geht dabei hervor, daß dadurch die Kraftübertragungsvorrichtung einmal um eine parallel zur Längsachse der Vorrichtung 1 ausgerichtete Achse drehbar ist, gleichzeitig aber auch um eine quer zur Längsachse der Vorrichtung 1 ausgerichtete Achse geschwenkt werden kann.

In Fig. 5 ist ein Befestigungsmittel 34 dargestellt, das eine Bohrung 9 besitzt, aber an dem Spannelement 2 über einen Zapfen 31 festgelegt werden kann, der in eine entsprechende Bohrung 32 am Spannelement eingreift.

Bei der Ausführungsform gemäß den Fig. 6 bis 8 ist ein Befestigungsmittel 35 vorgesehen, das im Bodenflansch 5 ein Langloch 33 besitzt, das mit Ausbuchtungen 37 ausgerüstet ist, die zur Aufnahme von Schraubenköpfen dienen.

Der Bodenflansch 5 schließt über ein Drehgelenk 38 an das Spannelement 2 an und kann dadurch, wie in Fig. 7 dargestellt, entsprechend abgewinkelt werden. Das Drehgelenk 38 wird bei dem dargestellten Ausführungsbeispiel, wie dies die Fig. 6 und 8 zeigen, durch eine Schraube gebildet, die gleichzeitig bei entsprechend festem Anziehen ein Verstarren des Gelenkes 38 ermöglicht.

Bei der Ausführungsform gemäß Fig. 9 ist zwar auch ein Langloch 33 vorgesehen, aber die Festlegung über eine Schraube erfolgt dabei über ein Rändelelement 39, dessen Zähne mit einer entsprechenden Verzahnung in der Bodenplatte 5 zusammenwirken können.

Fig. 1 zeigt, daß an das Spannelement 3 ein Rotationsbewegungen ermöglichender Körper 12 anschließen kann, der z. B. um eine Achse 11 drehbar ist, wobei die Achse 11 quer, d. h. im wesentlichen senkrecht zur Längsachse der Kraftübertragungsvorrichtung 1 ausgerichtet ist und quer, d. h. im wesentlichen senkrecht zur Längsachse des Haltemittels 7.

An den z. B. radartigen Körper 12 schließt eine Anschlußvorrichtung 14 an. Die erforderliche Drehbewegung des radartigen Körpers 12 um die Achse 11 erfolgt gemäß Fig. 1 über eine Schraubspindel 15, die sich einerseits ortsfest aber schwenkbar bei 21 an dem Spannelement 2 und andererseits ortsfest aber schwenkbar an der Außenseite des radartigen Körpers 12 bei 22 abstützt. In der Mitte der beiden Gewindespindelteile 40 und 41 ist eine Spindelmutter 23 angeordnet.

Es ist auch möglich, die Anschlußvorrichtung 14 drehbar in der Achse 11 zu lagern.

Gemäß der Ausführungsform entsprechend Fig. 2 wird eine Getriebespindel 16 eingesetzt, die sich drehbar und schwenkbar, aber ortsfest bei 21a an dem Spannelement 2 und anderenendes in einer mit Innengewinde ausgerüsteten Olive 22a abstützt. Das freie Ende der Spindel 16 ist mit einem Vierkant oder Mehrkant ausgerüstet, so daß hier ein Betätigungswerkzeug, z. B. ein Ratschenantrieb oder ein Schraubenradgetriebe angreifen kann.

Am Beispiel der Fig. 1 soll erläutert werden, daß es auch möglich ist, die Festlegung der Schraubspindel 15 bzw. der Getriebespindel 16 auch zusätzlich am Spannelement 3 vorzunehmen, so daß die eingestellte Winkelstellung auch bei Verstellung des Teiles 3 gegenüber dem Teil 2 beibehalten wird. Hierzu sind beispielsweise gemäß Fig. 1 an dem Spannelement 3 zusätzliche Befestigungsböckchen angeordnet, von denen das Befestigungsböckchen 50 erkennbar ist. Die gleiche Anordnung kann also auch bei der Einrichtung gemäß Fig. 2 vorgesehen werden.

Gemäß den Fig. 3 und 4 ist zusätzlich ein sich quer zur Längsachse der Kraftübertragungsvorrichtung 1 erstreckender Seitenarm 17 dargestellt, der bei der Darstellung in den Fig. 3 und 4 an das Spannelement 2 dadurch anschließt, daß an seinem entsprechenden Ende zwei Befestigungsflansche 24 und 25 vorgesehen sind, die über eine Befestigungsschraube 26 aneinander und an der Außenseite des Spannelementes 2 festgelegt werden. Um ein Kippen zu vermeiden, ist zusätzlich ein Distanzstück 27 eingesetzt. Ohne Einsatz des Distanzstückes 27 können die beiden Befestigungsflansche 24 und 25 auch an dem Spannelement 3 angeordnet und festgelegt werden.

An seinem anderen freien Ende trägt der Seitenarm 17 eine Anschlußvorrichtung 18 für eine Knochenschraube oder einen Gewindepin.

Der Seitenarm 17 ist zudem in der Länge verstellbar, indem beispielsweise das eine Armteil 28 eine Langlochbohrung 51 aufweist, durch die eine sich im anderen Armteil 29 abstützende Befestigungsschraube 52 greift.

In den Einrichtungen gemäß den Fig. 5 bis 9 ist im Spannelement 3 eine Öffnung für ein Betätigungsteil 43 vorgesehen, das an seinem unteren Ende mit einem Sechskant 44 ausgerüstet ist, der mit einem entsprechenden Innensechskant einer Knochenschraube od. dgl. zusammenwirken kann. Die in den verschiedenen Figuren dargestellten verschiedenen Ergänzungen und Einheiten können selbstverständlich miteinander kombiniert werden.

Aus den vorausgehenden Erläuterungen ist erkennbar, daß die beschriebenen Vorrichtungen Winkelkorrekturen in allen drei Ebenen des Raumes ermöglichen. Außerdem ist die Möglichkeit des Hebens und Senkens von Fragmenten gegeben. Die Vorrichtung arbeitet auch ohne Fixation an einer Platte, d. h. greift gegen verschiedene Widerlager an, wie Knochen, Schrauben usw. Durch die große allseitige Verstellbarkeit wird erreicht, daß die Vorrichtung an den Fixationselementen direkt am Kräftezentrum angreift, so daß Schraubenverbiegungen ausgeschlossen sind.

Schließlich ist darauf hinzuweisen, daß eine Durchführung der Fragmentverschiebung und Winkelkorrekturen über Getriebeeinrichtungen erfolgt, so daß eine wesentliche Arbeitserleichterung und bessere Dosierbarkeit erreicht wird.

## Patentansprüche

1. Kraftübertragungsvorrichtung bzw. Fixationsvorrichtung für osteosynthetische Arbeiten mit zwei relativ zueinander in Richtung der Längsachse der Kraftübertragungsvorrichtung (1) bewegbaren, über zugeordnete Haltemittel mittelbar oder unmittelbar am Knochen angreifenden Spannelementen (2, 3), zwischen denen eine Antriebsanordnung zur Erzeugung der Relativbewegung angeordnet ist, dadurch gekennzeichnet, daß wenigstens an einem der beiden in axialer Richtung gegeneinander verstellbaren Spannelementen (2, 3) ein um eine im wesentlichen rechtwinklig zur Verstellachse der beiden Spannelemente (2, 3) verlaufende Achse schwenkbares, kontrolliert antreibbares, aber feststellbares Bauteil (12) drehbar gelagert ist, das eine Anschlußvorrichtung (14) für eine Knochenschraube oder einen Knochenpin aufweist.

2. Vorrichtung nach Anspruch 1, dadurch gekennzeichnet, daß das Bauteil (12) zu seiner Schwenkbewegung mechanisch aktiv mittels eines Getriebes (15, 16) antreibbar ist.

3. Vorrichtung nach Anspruch 1 oder 2, dadurch gekennzeichnet, daß an einer Seite der Kraftübertragungsvorrichtung (1) ein Befestigungsmittel (4), z. B. ein Winkelstück, mit zwei rechtwinklig zueinander angeordneten Flanschen (5, 6) angeordnet ist und in Achsrichtung eine Bohrung (10) zur Aufnahme eines Befestigungsmittels (8) aufweist, so daß die Kraftübertragungsvorrichtung (1) um eine parallel zur Längsachse ausgerichtete Achse drehbar und/oder um eine im wesentlichen rechtwinklig zu dieser Längsachse ausgerichteten Achse schwenkbar ist.

4. Vorrichtung nach Anspruch 1 oder 2, gekennzeichnetdurch eine Schraubspindel (15) zum Antrieb des Bauteiles (12).

5. Vorrichtung nach Anspruch 1 oder 2, gekennzeichnet durch eine Getriebespindel (16) zum Antrieb des Bauteiles (12).

6. Vorrichtung nach Anspruch 1 oder 2, gekennzeichnet durch ein Schraubenrad oder Schneckengetriebe zum Antrieb des Bauteiles (12).

7. Vorrichtung nach einem oder mehreren der vorhergehenden Ansprüche, gekennzeichnet durch einen sich quer zur Längsachse der Kraftübertragungsvorrichtung (1) erstreckenden, an dem einen oder anderen Spannelement (2, 3) festlegbaren Seitenarm (17), an dessen freien Ende eine Anschlußvorrichtung (18) für eine Knochenschraube oder ein Gewindepin vorgesehen ist, z. B. zum Anheben oder Wegdrücken von Knochen oder Knochenfragmenten.

8. Vorrichtung nach Anspruch 7, dadurch gekennzeichnet, daß der Seitenarm (17) zweiteilig und damit längenverstellbar ausgebildet ist.

9. Vorrichtung nach einem der vorhergehenden Ansprüche, dadurch gekennzeichnet, daß das Bauteil (12) von dem Spannelement (3) um eine in Längsachse des Spannelementes (3) ausgerichtete Achse drehbar und feststellbar getragen ist.

10. Vorrichtung nach einem der vorhergehenden Ansprüche, dadurch gekennzeichnet, daß der Seitenarm (17) auch unmittelbar an dem Spannelement (2) und/oder an dem Spannelement (3) angeordnet ist.

11. Vorrichtung nach einem der vorhergehenden Ansprüche, dadurch gekennzeichnet, daß die Schraubspindel (15) bzw. die Getriebespindel (16) abnehmbar an dem Spannelement (2) bzw. (3) angeordnet sind.

12. Vorrichtung nach einem der vorhergehenden Ansprüche, dadurch gekennzeichnet, daß an einer Seite der Kraftübertragungsvorrichtung ein Befestigungsmittel (34) vorgesehen ist, das eine Bohrung (9) zur Aufnahme eines Haltemittels aufweist und über einen Zapfen (31) an einer Bohrung (32) des Spannelementes (2) festgelegt werden kann.

13. Vorrichtung nach einem der vorhergehenden Ansprüche, dadurch gekennzeichnet, daß an einer Seite der Kraftübertragungsvorrichtung (1) ein Befestigungsmittel (35) oder (36) vorgesehen ist, das eine Bodenplatte (5) aufweist, in der ein Langloch (33) zur Aufnahme eines Haltemittels vorgesehen ist und das schwenkbar über ein Drehgelenk (38) mit einer quer zur Längsachse des Spannelementes (3) ausgerichteten Achse an das Spannelement (2) anschließt.

14. Vorrichtung nach Anspruch 2, dadurch gekennzeichnet, daß das Getriebeteil zum Antrieb des Bauteiles (12) auch an dem Spannelement (3) festlegbar ist (Befestigungsböckchen 40).

15. Vorrichtung nach einem der vorhergehenden Ansprüche, dadurch gekennzeichnet, daß die Befestigungsöffnung in dem Bodenflansch (5) des Befestigungsmittels (35) bzw. (36) mit einer Verrasterung für das Befestigungsmittel ausgerüstet ist.

## Claims

1. A load transmission device or fixing device for osteosynthetic work comprising two tension elements (2, 3) acting indirectly or directly on the bone via associated holding means and movable relative to each other in the direction of the longitudinal axis of the load transmission device (1), between which tension elements (2, 3) there is arranged a drive arrangement for generating the relative movement, characterized in that there is mounted rotatably at least on one of the two tension elements (2, 3) adjustable with respect to each other in the axial direction a controlledly drivable but lockable component (12) which is swivellable about an axis running substantially at right angles to the adjusting axis of the two tension elements (2, 3) and which comprises a connecting device (14) for a bone screw or a bone pin.

2. A device according to claim 1, characterized in that the component (12) may be driven into its swivel movement mechanically actively by means of gearing (15, 16).

3. A device according to claim 1 or claim 2, characterized in that a fastening means (4), e.g. an angle piece, with two flanges (5, 6) disposed at right angles to each other is arranged on one side of the load transmission device (1) and comprises in the axial direction a bore (10) for receiving a fastening means (8), such that the load transmission device (1) may be rotated about an axis oriented parallel to the longitudinal axis and/or may be swivelled about an axis oriented substantially at right angles to this longitudinal axis.

4. A device according to claim 1 or claim 2, characterized by a screw spindle (15) for driving the component (12).

5. A device according to claim 1 or claim 2, characterized by a gear spindle (16) for driving the component (12).

6. A device according to claim 1 or claim 2, characterized by a screw wheel or worm gear for driving the component (12).

7. A device according to any one or more of the preceding claims, characterized by a side arm (17) extending perpendicularly to the longitudinal axis of the load transmission device (1) and fastenable to one or other of the tension elements (2, 3), at the free end of which side arm (17) there is provided a connecting device (18) for a bone screw or a threaded pin, e.g. for lifting or pushing away bone or bone fragments.

8. A device according to claim 7, characterized in that the side arm (17) is of two part and therefore length-adjustable construction.

9. A device according to any one of the preceding claims, characterized in that the component (12) is carried by the tension element (3) so as to be fastenable and rotatable about an axis directed along the longitudinal axis of the tension element (3).

10. A device according to any one of the preceding claims, characterized in that the side arm (17) is also arranged directly on the tension element (2) aand/or on the tension element (3).

11. A device according to any one of the preceding claims, characterized in that the screw spindle (15) and the gear spindle (16) are arranged removably on the tension element (2) or (3), respectively.

12. A device according to any one of the preceding claims, characterized in that on one side of the load transmission device there is provided a fastening means (34) which comprises a bore (9) for receiving a holding means and which may be fixed to a bore (32) in the tension element (2) via a pin (31).

13. A device according to any one of the preceding claims, characterized in that on one side of the load transmission device (1) there is provided a fastening means (35) or (36) which comprises a base plate (5) in which there is provided an elongate hole (33) for receiving a holding means and which is swivellably connected to the tension element (2) via a hinge (38) with an axis oriented perpendicularly to the longitudinal axis of the tension element (3).

14. A device according to claim 2, characterized in that the gear member for driving the component (12) may also be fixed to the tension element (3) (fastening bracket (50)).

15. A device according to any one of the preceding claims, characterized in that the fastening aperture in the base flange (5) of the fastening means (35) or (36) is equipped with a catch for the fastening means.

## Revendications

1. Dispositif de transmission de force et/ou dispositif de fixation pour effectuer des travaux d'ostéosynthèse, comprenant deux éléments de serrage (2, 3) pouvant être déplacés, en mouvement relatif l'un par rapport à l'autre, dans la direction de l'axe longitudinal du dispositif de transmission de force (1) et agissant, par l'intermédiaire de moyens de fixation associés, directement ou indirectement sur l'os, un équipement d'entraînement produisant le mouvement relatif étant disposé entre lesdits éléments de serrage, caractérisé par un élément (12) pouvant tourner autour d'un axe orienté dans un angle sensiblement perpendiculaire par rapport à l'axe de réglage des deux éléments de serrage (2, 3) et pouvant être entraîné, mais également verrouillé, ledit élément (12) présentant un dispositif de raccordement (14) pour une vis ou un clou d'ostéosynthèse.

2. Dispositif selon la revendication 1, caractérisé en ce que l'élément (12) peut être mis en mouvement rotatif par un entraînement mécanique par l'intermédiaire d'un engrenage (15, 16).

3. Dispositif selon la revendication 1 ou 2, caractérisé en ce qu'un moyen de fixation (4), par exemple un cornière, est disposé sur l'une des faces du dispositif de transmission de force (1), ledit moyen de fixation présentant deux brides (5, 6) disposées perpendiculairement l'une par rapport à l'autre et présentant un alésage (10) dans le sens axial pour recevoir un moyen de fixation (8), de sorte que le dispositif de transmission de force (1) puisse être tourné autour d'un axe parallèle à l'axe longitudinal et/ou autour d'un axe sensiblement perpendiculaire à cet axe longitudinal.

4. Dispositif selon la revendication 1 ou 2, caractérisé par une vis sans fin (15) utilisée pour entraîner l'élément (12).

5. Dispositif selon la revendication 1 ou 2, caractérisé par une vis de transmission (16) utilisée pour entraîner l'élément (12).

6. Dispositif selon la revendication 1 ou 2, caractérisé par une roue à dents hélicoïdale ou par un engrenage à vis sans fin utilisé pour entraîner l'élément (12).

7. Dispositif selon l'une ou plusieurs des revendications précédentes, caractérisé par un bras latéral (17) orienté transversalement à l'axe longitudinal du dispositif de transmission de force (1), d'une part, et pouvant être fixé sur l'un ou l'autre des éléments de serrage (2, 3), d'autre part, un dispositif de raccordement (18) pour une vis d'ostéosynthèse ou un boulon fileté étant prévu à l'extrémité libre dudit bras latéral pour soulever ou écarter des os ou des fragments d'os.

8. Dispositif selon la revendication 7, caractérisé en ce que le bras latéral est composé de deux éléments de façon à pouvoir être réglé en longueur.

9. Dispositif selon l'une quelconque des revendications précédentes, caractérisé en ce que l'élément (12) est maintenu par l'élément de serrage (3) de manière à pouvoir être tourné autour d'un axe orienté dans le sens de l'axe longitudinal de l'élément de serrage (3) et verrouillé.

10. Dispositif selon l'une quelconque des revendications précédentes, caractérisé en ce que le bras latéral (17) est également disposé directement sur l'élément de serrage (2) et/ou sur l'élément de serrage (3).

11. Dispositif selon l'une quelconque des revendications précédentes, caractérisé en ce que la vis sans fin (15) ou la vis de transmission(16)est disposée de manière amovible sur l'élément de serrage (2) ou (3).

12. Dispositif selon l'une quelconque des revendications précédentes, caractérisé en ce qu'un moyen de fixation (34) est prévu sur l'une des faces du dispositif de transmission de force, ledit moyen de fixation présentant un alésage (9) pour loger un moyen de fixation, d'une part, et pouvant être fixé, par un tourillon (31), dans un alésage (32) aménagé dans l'élément de serrage (2), d'autre part.

13. Dispositif selon l'une quelconque des revendications précédentes, caractérisé en ce qu'un moyen de fixation (35) ou (36) est prévu sur l'une des faces du dispositif de transmission de force (1), ledit moyen de fixation présentant une plaque de base (5) dans laquelle est aménagé un trou oblong (33) pour loger un moyen de fixation, d'une part, et relié à l'élément de serrage (2) de façon à pouvoir pivoter, par une articulation à charnière (38) dont l'axe est orienté transversalement par rapport à l'axe longitudinal de l'élément de serrage (3), d'autre part.

14. Dispositif selon la revendication 2, caractérisé en ce que l'élément d'engrenage utilisé pour entraîner l'élément (12) peut également être fixé sur l'élément de serrage (3) (patte d'attache 50).

15. Dispositif selon l'une quelconque des revendications précédentes, caractérisé en ce que l'ouverture de fixation prévue dans la bride de fond (5) du moyen de fixation (35) ou (36) est munie d'un crantage pour le moyen de fixation.
